# EUROPÄISCHE PATENTSCHRIFT

(11) **EP 1 901 655 B1**
(45) Veröffentlichungstag und Bekanntmachung des Hinweises auf die Patenterteilung: **19.12.2018**
(21) Anmeldenummer: 06754473.4
(22) Anmeldetag: 21.06.2006
(51) Int. Cl.: A61B 5/151

(54) **LANZETTENVORRICHTUNG**
LANCET DEVICE
DISPOSITIF A LANCETTE

(30) Priorität: 13.07.2005 EP 05015171; 03.03.2006 EP 06004319
(43) Veröffentlichungstag der Anmeldung: 26.03.2008
(73) Patentinhaber: Roche Diabetes Care GmbH, 68305 Mannheim (DE); F. Hoffmann-La Roche AG, 4070 Basel (CH)
(72) Erfinder: DECK, Frank, 67150 Niederkirchen (DE); HOERAUF, Christian, 68723 Oftersheim (DE); HINRICHS, Malte, 10437 Berlin (DE); GRUNWALD, Torsten, 23714 Malente (DE); JOTTER, Mathias, 23701 Eutin (DE); RUFER, Manfred, 23715 Bosau (DE); STORJOHANN, Jens, 22765 Hamburg (DE); BERG, Hartmut, 23714 Malente (DE)
(74) Vertreter: Riwotzki, Karsten
(86) Internationale Anmeldenummer: PCT/EP2006/005929
(87) Internationale Veröffentlichungsnummer: WO 2007/006399

(56) Entgegenhaltungen:
- WO-A2-02/100460
- JP-A- 2 095 352
- JP-A- 2003 339 680
- US-A- 6 080 172

## Beschreibung

Die Erfindung betrifft eine Lanzettenvorrichtung, mit der eine Lanzette entlang eines Einstichweges zum Erzeugen einer Einstichwunde in einer Hautoberfläche, insbesondere zum Gewinnen von Körperflüssigkeit für Diagnosezwecke, verschiebbar ist, umfassend einen Lanzettenantrieb mit einem Antriebsmittel zum Erzeugen einer Antriebskraft für eine Einstichbewegung der Lanzette entlang des Einstichweges in Richtung auf die Hautoberfläche, wobei der Lanzettenantrieb einen Permanentmagneten umfasst, mit dem eine der Antriebskraft entgegen gerichtete magnetische Haltekraft erzeugbar ist, und der Lanzettenantrieb ferner Auslösemittel umfasst, mit denen die Haltekraft soweit reduzierbar ist, dass die Lanzette unter Einwirkung der von dem Antriebsmittel erzeugten Antriebskraft in Richtung auf die Hautoberfläche beschleunigt wird.

Lanzettenvorrichtungen werden beispielsweise von Diabetikern benötigt, die ihren Blutzuckerspiegel häufig kontrollieren müssen, um ihn durch Insulininjektionen innerhalb bestimmter Sollgrenzen halten zu können. Durch umfangreiche wissenschaftlichen Untersuchungen wurde bewiesen, dass mittels einer Intensivtherapie mit mindestens vier Analysen pro Tag eine dramatische Reduzierung schwerster Spätschäden des Diabetes mellitus (beispielsweise eine Retinopathie mit resultierender Erblindung des Patienten) erreicht werden kann.

Für Benutzer von Lanzettenvorrichtungen ist einerseits ein möglichst schmerzarmer Einstich und andererseits eine möglichst einfache Bedienung und Handhabbarkeit des verwendeten Lanzettengeräts von großer Bedeutung.

Voraussetzung für einen schmerzarmen Einstich ist eine möglichst schnelle Einstichbewegung mit einer kurzen Verweildauer der Lanzette in der Haut. Im Stand der Technik hat sich für eine entsprechend starke Beschleunigung der Lanzetten die Verwendung von Antriebsfedern bewährt. Ein Nachteil derartiger Lanzettenvorrichtungen liegt darin, dass ein manuelles Spannen der Antriebsfeder nach einem erfolgten Einstich für viele Benutzer mühsam ist. Dies gilt insbesondere für Personen, deren manuelle Geschicklichkeit durch Alter oder Krankheit eingeschränkt ist.

Eine Lanzettenvorrichtung, bei der die Antriebsfeder durch einen Elektromotor automatisch gespannt wird, bietet in dieser Hinsicht zwar einen erhöhten Benutzerkomfort, hat jedoch den Nachteil wegen des Elektromotors größer und schwerer zu sein. Eine Lanzettenvorrichtung mit einem integrierten Elektromotor stellt deshalb für Benutzer, die diese für häufige Messungen ständig mit sich führen müssen, eine Belastung dar. Hinzu kommt, dass die Herstellungskosten durch einen Elektromotor wesentlich erhöht werden.

Im Stand der Technik sind ferner Lanzettenvorrichtungen bekannt, bei denen die Antriebskraft auf elektromagnetische Weise mittels einer Spule erzeugt wird. Derartige Lanzettenvorrichtungen sind beispielsweise in der WO 02/100460 A2 und in der US 6364889 B1 offenbart. Um mit derartigen elektromagnetischen Lanzettenantrieben eine für einen schmerzarmen Einstich ausreichend schnelle Einstichbewegung zu bewirken, müssen starke Magnetfelder erzeugt werden. Dies erfordert, dass durch die verwendeten Antriebsspulen relativ starke elektrische Ströme fließen, die sich in einem kleinen, kompakten Handgerät nicht oder nur mit großem Aufwand erzeugen lassen. Elektromagnetische Lanzettenantriebe konnten sich deshalb bisher nicht gegen mechanische Antriebe mit Antriebsfedern durchsetzen.

Das Dokument JP 2003 339680 A offenbart eine entsprechende Lanzettenvorrichtung, wobei der Lanzettenantrieb einen Permanentmagneten umfasst, mit dem eine der Antriebskraft entgegen gerichtete magnetische Haltekraft erzeugbar ist, und der Lanzettenantrieb ferner Auslösemittel umfasst, mit denen die Haltekraft soweit reduzierbar ist, dass die Lanzette unter Einwirkung der von dem Antriebsmittel erzeugten Antriebskraft in Richtung auf die Hautoberfläche beschleunigt wird.

Aufgabe der Erfindung ist es, einen kostengünstigen Weg aufzuzeigen, wie bei einer Lanzettenvorrichtung der eingangs genannten Art mit einem kompakten Design eine für einen schmerzarmen Einstich ausreichend schnelle Einstichbewegung erzeugt und der Benutzer von Vorbereitungshandlungen, wie beispielsweise dem Spannen einer Antriebsfeder, möglichst weitgehend entlastet werden kann.

Diese Aufgabe wird mit einer Lanzettenvorrichtung der eingangs genannten Art erfindungsgemäß dadurch gelöst, dass das Antriebsmittel einen weiteren Permanentmagneten umfasst, der dem die Haltekraft erzeugenden Permanentmagneten entgegengesetzt polarisiert angeordnet ist.

Als Antriebsmittel kann eine Antriebsfeder verwendet werden, die durch die magnetische Haltekraft in einem gespannten Zustand gehalten werden kann. Bei einer erfindungsgemäßen Lanzettenvorrichtung kann die Lanzette nach dem Eindringen in die Hautoberfläche mittels der magnetischen Haltekraft in ihre Ausgangsposition zurück gezogen werden. Die Haltekraft kann beispielsweise mit einem Elektromagneten erzeugt werden oder - erfindungsgemäß - von einem Permanentmagneten ausgehen. Wird ein Permanentmagnet verwendet, so ist es vorteilhaft, wenn die Auslösemittel eine Spule umfassen, mit der ein Magnetfeld erzeugbar ist, das die Haltekraft des Permanentmagneten zumindest teilweise, vorzugsweise vollständig, kompensiert. Durch geeignete Dimensionierung des Permanentmagneten und der Spule lässt sich dabei erreichen, dass die von dem Magneten erzeugte Haltekraft ausreichend groß ist, um nach einem erfolgten Einstich ein erneutes Spannen der Antriebsfeder zu bewirken.

Eine Lanzettenvorrichtung mit einer Antriebsfeder hat den Vorteil, dass sie wesentlich kostengünstiger und kompakter gefertigt werden kann, als eine Lanzettenvorrichtung mit einem Elektromotor, und dennoch ein selbsttätiges Spannen der Antriebsfeder ermöglicht.

Als Antriebsmittel für eine erfindungsgemäße Lanzettenvorrichtung wird eine Spule verwendet, um die Antriebskraft auf magnetischem Wege zu erzeugen. Die Spule kann zugleich auch als Auslösemittel verwendet werden, mit dem die magnetische Haltekraft überkompensiert wird. Die magnetische Haltekraft wird von einem Permanentmagneten erzeugt, dem als zweiter Teil des Antriebsmittels ein weiterer Permanentmagnet als Antriebsmagnet mit umgekehrter Polarisierung zugeordnet ist, so dass sich die Magnetfelder der Permanentmagnete destruktiv überlagern. Auf diese Weise wird die von dem Antriebsmagneten erzeugte Antriebskraft von dem die Haltekraft erzeugenden Permanentmagneten kompensiert, so dass sich ohne Spulenstrom keine resultierende Antriebskraft und folglich auch keine Lanzettenbewegung ergibt. Fließt durch die Antriebsspule ein Strom, überlagert sich das Magnetfeld des Antriebsmagneten konstruktiv mit dem Magnetfeld der Spule, so dass eine resultierende Antriebskraft zur Beschleunigung einer Lanzette entsteht.

Selbst wenn sich die beiden Permanentmagnete exakt kompensieren, kann verblüffenderweise durch die Verwendung einer Antriebsspule in Kombination mit entgegen gesetzten polarisierten Permanentmagneten eine größere Antriebskraft erzeugt werden als mit einer Antriebsspule für sich alleine. Durch die Überlagerung des Spulenfeldes mit den Feldern der entgegen gesetzt polarisierten Permanentmagneten ergibt sich nämlich lokal in einem ersten Bereich eine erhöhte magnetische Feldstärke und in einem zweiten Bereich eine lokal reduzierte Feldstärke. Die lokal erhöhte Magnetfeldstärke kann dazu benutzt werden, ein Antriebselement, beispielsweise einen weichmagnetischen Spulenkern, zu magnetisieren. Die von dem Magnetfeld auf das Antriebselement ausgeübte Kraft ist dann wegen der lokal erhöhten Feldstärke insgesamt größer als bei Verwendung einer Spule ohne Permanentmagnete. Ein wichtiger Aspekt der Erfindung, der auch selbstständig die Bedeutung haben kann, betrifft deshalb eine Lanzettenvorrichtung umfassend einen Lanzettenantrieb mit:
- einer ersten und einer zweiten Magnetfeldquelle, die im Betrieb zwei sich in einem Antriebsraum destruktiv überlagernde Magnetfelder erzeugen,
- einer dritten Magnetfeldquelle, die im Betrieb ein weiteres Magnetfeld erzeugt, das sich in dem Antriebsraum konstruktiv mit dem Magnetfeld der ersten Magnetfeldquelle und destruktiv mit dem Magnetfeld der zweiten Magnetfeldquelle überlagert, wobei mindestens eine der drei Magnetfeldquellen ein Permanentmagnet und mindestens eine der drei Magnetfeldquellen eine Spule ist, und
- einem Antriebselement in Form eines weichmagnetischen Spulenkerns, der in dem Antriebsraum zwischen einer ersten und einer zweiten Position hin und her beweglich ist, um durch Kopplung mit einer Lanzette eine Einstich- und Rückführbewegung zu bewirken, wobei die Magnetisierung des Spulenkerns von der dritten Magnetfeldquelle bestimmt wird, so dass der Spulenkern durch einen Spulenstrom aus der ersten in die zweite Position und durch Umkehrung der Richtung des Spulenstroms zurück in die erste Position bewegbar ist.

Die Erfindung wird nachfolgend anhand eines in den Figuren dargestellten Ausführungsbeispiels näher erläutert. Die dargestellten Besonderheiten können einzeln oder in Kombination verwendet werden, um bevorzugte Ausgestaltungen der Erfindung zu schaffen. Es zeigen:
- Fig. 1: eine Lanzettenvorrichtung mit gespannter Antriebsfeder;
- Fig. 2: die in Fig. 1 gezeigte Lanzettenvorrichtung mit entspannter Antriebsfeder;
- Fig. 3: eine weitere Darstellung der Lanzettenvorrichtung von Fig. 1;
- Fig. 4: ein Ausführungsbeispiel eines Lanzettenantriebs einer erfindungsgemäßen Lanzettenvorrichtung in einer teilweise geschnittenen Schrägansicht;
- Fig. 5: den Feldverlauf der von den Permanentmagneten des in Figur 4 dargestellten Lanzettenantriebs erzeugten Magnetfelder;
- Fig. 6: den Feldverlauf des im Betrieb von den Spulenwicklungen des in Figur 4 dargestellten Lanzettenantriebs erzeugten Magnetfelds und
- Fig. 7: den Verlauf des Gesamtfeldes, das sich durch Überlagerung der in den Figuren 5 und 6 dargestellten Magnetfelder ergibt.

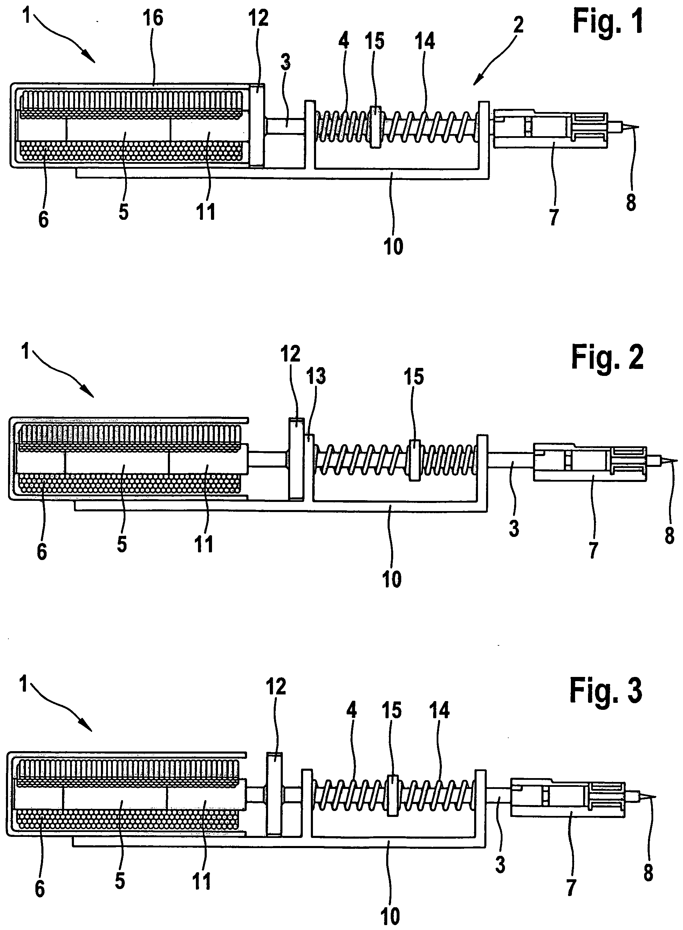

Die in den Figuren 1 bis 3 dargestellte, nicht vollständig erfindungsgemäß ausgebildete Lanzettenvorrichtung kann beispielsweise in ein Analysehandgerät integriert werden, das eine Messeinrichtung zur Untersuchung von Körperflüssigkeit, die aus einer erzeugten Einstichwunde gewonnen wird, aufweist. Ebenso gut kann die dargestellte Lanzettenvorrichtung in ein separates Gerät als Stechhilfe eingebaut werden.

Zentraler Bestandteil der dargestellten Lanzettenvorrichtung 1 ist ein Lanzettenantrieb 2, der eine Schubstange 3, ein Antriebsmittel in Form einer Antriebsfeder 4, eine Spule 6 und einen axial in der Spule 6 angeordneten Permanentmagneten 5 umfasst. Die Schubstange 3 trägt einen Lanzettenhalter 7 mit einer austauschbaren Lanzette 8 und wird zum Erzeugen einer Einstichwunde mit der als Schraubenfeder ausgebildeten Antriebsfeder 4 entlang eines durch die Führung 10 vorgegebenen Einstichwegs beschleunigt.

In Fig. 1 ist die Schubstange 3 in ihrer Ruheposition mit gespannter Antriebsfeder 4 dargestellt. Die Schubstange 3 wird in dieser Position durch die magnetische Haltekraft des in den Spulenkern 11 der Spule 6 integrierten Permanentmagneten 5 gehalten. Der Spulenkern 11 mit dem Permanentmagneten 5 ist ortsfest hinsichtlich der Spule 6 angeordnet. Die Spule 6 dient als Auslösemittel zum Auslösen einer Einstichbewegung. Indem man durch die Spule 6 einen elektrischen Strom fließen lässt, wird ein Magnetfeld erzeugt, das die Haltekraft des Permanentmagneten 5 kompensiert, so dass sich die Antriebsfeder 4 entspannt und die Schubstange 3 mit der Lanzette 8 unter Einwirkung der dabei von der Antriebsfeder 4 erzeugten Antriebskraft in Richtung auf die Hautoberfläche beschleunigt wird.

Die Schubstange 3 ist bevorzugt aus Kunststoff und trägt eine Ankerplatte 12 aus Eisen oder einem anderen ferromagnetischen Material, mit der in der in Figur 1 gezeigten Position ein magnetischer Kreis, der den Permanentmagneten 5 und einen die Spule 6 umgebenden Polschuh 16 enthält, geschlossen wird.

Die Ankerplatte 12 kann auch als Permanentmagnet ausgebildet sein, so dass sie nicht nur von dem Permanentmagneten 5 angezogen, sondern auch mit einem von der Spule erzeugten Magnetfeld abgestoßen und zusätzlich beschleunigt werden kann.

Ein Vergleich der Fig. 1 und 2 zeigt, dass die Schubstange 3 in der Ruheposition mit einem Abschnitt in den Spulenkern 11 hineinragt. Auf diese Weise wird eine verbesserte Führung erreicht.

In Fig. 2 ist die Lanzettenvorrichtung 1 mit der Schubstange 3 in der Einstichposition dargestellt. In der Einstichposition liegt ein von der Ankerplatte 12 gebildeter Anschlag an einem Begrenzungselement 13 der Führung 10 an, so dass der Einstichweg begrenzt wird.

Der Lanzettenantrieb 2 umfasst neben der Antriebsfeder 6 ferner eine Rückholfeder 14 zum Erzeugen einer Rückführbewegung der Lanzette 8. Mit der Rückführbewegung wird die Antriebsfeder 4 erneut gespannt. Antriebsfeder 4 und Rückholfeder 14 sind jeweils als Schraubenfedern ausgebildet, welche die Schubstange 3 umgeben. Die Antriebsfeder 4 und die Rückholfeder 14 stützen sich jeweils mit einem Ende an einem Stützabschnitt 15 der Schubstange 3, der bei der dargestellten Lanzettenvorrichtung 1 als Verdickung ausgeführt ist, und mit dem jeweils anderen Ende an der Führung 10 ab. Die Antriebsfeder 4 und die Rückholfeder 14 sind so angeordnet, dass ein Entspannen der Antriebsfeder 4 ein Spannen der Rückholfeder 14 und ein Entspannen der Rückholfeder 14 ein Spannen der Antriebsfeder 4 bewirkt.

Der Begriff "spannen" ist in diesem Zusammenhang so zu verstehen, dass in der jeweils betroffenen Feder Energie gespeichert wird. Dies kann beispielsweise bei einer Kompressionsfeder durch Zusammendrücken und bei einer Dehnungsfeder durch Auseinanderziehen bewirkt werden.

Selbstverständlich treten bei der dargestellten Lanzettenvorrichtung 1 Reibungskräfte auf, so dass die in der Rückholfeder 14 in der in Fig. 2 dargestellten Einstichposition gespeicherte Energie nicht ganz zum erneuten Spannen der Antriebsfeder 4 ausreicht. Bei der dargestellten Vorrichtung wird die Rückholfeder 14 deshalb bei der Rückführbewegung der Schubstange 3 von dem Permanentmagneten 5 unterstützt. Die von dem Permanentmagneten 5 erzeugte Haltekraft und die von der Rückholfeder 14 zu Verfügung gestellte Federkraft reichen bei einer entsprechenden Auslegung des Permanentmagneten 5 aus, um die Schubstange 3 erneut in die in Fig. 1 dargestellte Ruheposition zu bringen und dabei die Antriebsfeder 4 zu spannen. Durch eine geeignete Formgebung der Enden des Polschuhs 16, beispielsweise durch Abschrägen der Enden, kann die Kraft-Wegcharakteristik der mit dem Permanentmagneten erzeugten Haltekraft beeinflusst und die Rückführbewegung zusätzlich unterstützt werden.

Damit die Haltekraft des Permanentmagneten 5 für ein erneutes Spannen der Antriebsfeder 4 genutzt werden kann, genügt es den zum Auslösen eines Einstichs durch die Spule 6 geschickten Strom abzuschalten. Sobald durch die Spule 6 kein Strom mehr fließt, wird von der Spule 6 auch kein Magnetfeld mehr erzeugt, so dass die Haltekraft des Permanentmagneten 5 nicht mehr kompensiert wird und sich zu der Federkraft der Rückholfeder 14 addiert.

Eine Einstich- und Rückführbewegung der Lanzette dauert insgesamt typischerweise 4 ms bis 6 ms. Damit die Haltekraft des Permanentmagneten für ein erneutes Spannen der Antriebsfeder genutzt werden kann, wird deshalb bevorzugt der zum Auslösen eines Einstichs durch die Spule 6 geschickte Strom nach 1 ms bis 3 ms, bevorzugt 1,5 ms bis 2,5 ms, abgeschaltet. Besonders gut ist ein Strompuls mit möglichst steilen Flanken, idealerweise einem Rechteckprofil, geeignet.

Für eine möglichst schnelle Einstichbewegung ist eine entsprechend starke Antriebsfeder 4 und ein entsprechend starker Permanentmagnet 5, beispielsweise ein Seltenerdmagnet, bevorzugt. Zur Kompensation der magnetischen Haltekraft werden deshalb bevorzugt Spannungen und/oder Stromstärken verwendet, die weit über die Leistungsfähigkeit handelsüblicher Batterien hinaus gehen. Bevorzugt ist die Spule 6 deshalb über Strompuffer und/oder Spannungswandler an eine interne Stromquelle der Lanzettenvorrichtung, beispielsweise eine Batterie, angeschlossen, so dass mit handelsüblichen Batterien ein zum Kompensieren der magnetischen Haltekraft geeigneter Strompuls erzeugt werden kann. Als Strompuffer sind beispielsweise Kondensatoren oder Akkumulatoren, insbesondere Lithium-Polymer-Akkumulatoren, geeignet. Geeignete Spannungswandler sind als DC/DC-Konverter verfügbar. Die entsprechende Technologie zum Erzeugen intensiver Strompulse ist beispielsweise zum Erzeugen von Lichtblitzen bei Fotoapparaten gebräuchlich und kann für die beschriebene Lanzettenvorrichtung genutzt werden.

Um die Rückführbewegung zusätzlich zu unterstützen, kann die Richtung des zum Auslösen eines Einstichs durch die Spule 6 fließenden Stroms umgepolt werden, so dass sich das von der Spule 6 erzeugte Magnetfeld zu der Haltekraft des Permanentmagneten 5 addiert. Zum Umpolen des Stroms kann beispielsweise eine Steuereinheit mit einer H-Brücke verwendet werden. Idealerweise erfolgt das Umpolen in dem Moment, in dem die Lanzette den äußersten Punkt des Einstichwegs erreicht hat.

Eine weitere Möglichkeit, um die Schubstange 3 in einem Störfall aus einer in Fig. 3 dargestellten Zwischenposition wieder in die Ruheposition zu bewegen, besteht darin, mittels der Spule 6 durch periodische Stromstöße eine mechanische Schwingung des von der Antriebsfeder 4 der Rückholfeder 14 und der Schubstange 3 gebildeten mechanischen Systems anzuregen. Bei fortwährender Anregung mit der Resonanzfrequenz dieses Systems nehmen die Amplituden dieser Schwingung zu, bis die Schubstange 3 in die Ruheposition zurückkehrt und dort von der Haltekraft des Permanentmagneten 5 gehalten werden kann.

Durch eine Messung der Induktivität der Spule 6 kann festgestellt werden, ob die Ankerplatte 12 an dem Polschuh 16 anliegt. Auf diese Weise kann also ermittelt werden, ob sich die Schubstange 3 in der in Figur 1 dargestellten Ruheposition befindet oder nicht. Bevorzugt wird kurz nach einem Einstich, beispielsweise 1 bis 2 Sekunden nach einem Einstich, eine Messung der Induktivität der Spule 6 durchgeführt. Falls dabei festgestellt wird, dass sich die Schubstange 3 nicht in der Ruheposition befindet, wird durch periodische Stromstöße eine mechanische Schwingung des von der Antriebsfeder 4, der Rückholfeder 14 und der Schubstange 3 gebildeten mechanischen Systems angeregt, so dass die Schubstange 3 in ihre Ruheposition zurückkehrt.

Die Spule 6 wird auf diese Weise als Positionssensor für die Position der Schubstange 3 verwendet. Die dargestellte Lanzettenvorrichtung kann alternativ oder zusätzlich auch mit anderen Positionssensoren ausgerüstet werden, so dass der optimale Zeitpunkt zum Abschalten oder Umpolen des Stroms durch die Spule 6 in Abhängigkeit von der momentanen Position der Schubstange 3 ermittelt werden kann. Der Einsatz von Sensoren ermöglicht deshalb anstelle einer einfachen Steuerung des Spulenstroms, bei dem ein vorgegebenes Profil für den Strompuls vorgegeben wird, eine Regelung des Spulenstroms in Abhängigkeit von der Position der Schubstange 3.

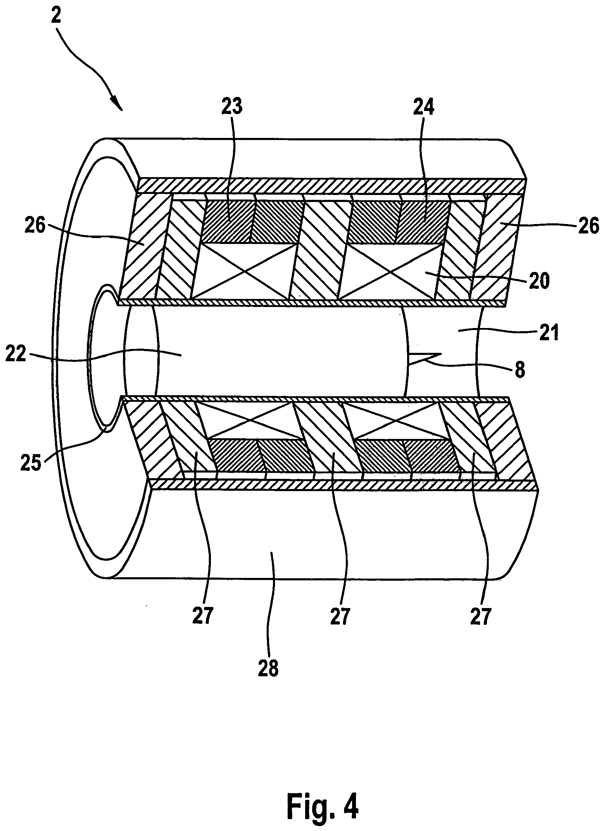

In Figur 4 ist ein Ausführungsbeispiel eines erfindungsgemäßen Lanzettenantriebs 2 für eine Lanzettenvorrichtung dargestellt. Bei dem in Figur 4 dargestellten Ausführungsbeispiel wird als Antriebsmittel zum Erzeugen des Antriebskraft eine Spule 20 verwendet, die einen Antriebsraum 21 umgibt, in dem ein Antriebselement in Form eines weichmagnetischen Spulenkerns 22 hin und her beweglich ist, um durch Kopplung mit einer Lanzette, beispielsweise mittels einer in den Figuren 1 bis 3 dargestellten Schubstange 3, eine Einstich- und Rückführbewegung der Lanzette zu bewirken. Der Antriebsraum 21 ist ferner von Permanentmagneten 23, 24 umgeben, die bei dem dargestellten Ausführungsbeispiel als Magnetringe ausgebildet sind. Idealerweise sind die Permanentmagnetringe 23, 24 konzentrisch mit der Spule 20 angeordnet.

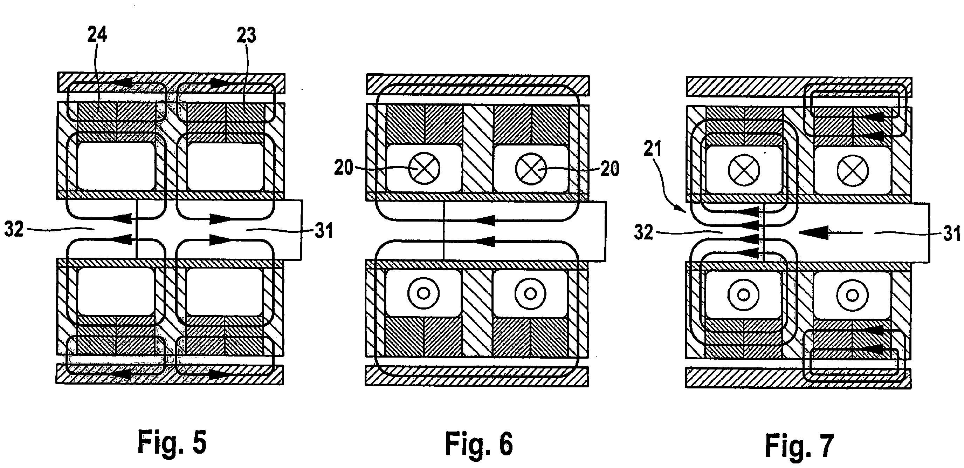

Wie der in Figur 5 dargestellte Feldverlauf des von den Permanentmagneten 23, 24 erzeugten Magnetfelds zeigt, sind die Permanentmagnete 23, 24 entgegengesetzt polarisiert angeordnet. Die Permanentmagnete 23, 24 stellen also eine erste und eine zweite Magnetfeldquelle dar, die zwei sich in dem Antriebsraum 21 destruktiv überlagernde Magnetfelder erzeugen. Idealerweise sind die beiden Permanentmagnete 23, 24 gleich und lediglich unterschiedlich angeordnet. Bevorzugt unterschieden sich die Permanentmagnete 23, 24 in ihrer Stärke um weniger als 30 %, besonders bevorzugt um weniger als 20 %, insbesondere um weniger als 10 %.

Die Permanentmagnete 23, 24 wirken mit der Spule 20 zusammen, die als dritte Magnetfeldquelle im Betrieb ein weiteres Magnetfeld erzeugt, dessen Feldverlauf in Figur 6 dargestellt ist. Zum Auslösen eines Einstichs lässt man einen elektrischen Strom durch die Windungen der Spule 20 fließen, so dass sich das in Figur 7 dargestellte magnetische Gesamtfeld ergibt. Wie man darin erkennt, überlagert sich das von der Spule erzeugte Magnetfeld in dem Antriebsraum 21 konstruktiv mit dem Magnetfeld des Permanentmagneten 24 und destruktiv mit dem Magnetfeld des Permanentmagneten 23. Auf diese Weise ergibt sich in dem Antriebsraum 21 ein Gesamtfeld, das in einem ersten Teilbereich 31 durch destruktive Überlagerung weitgehend reduziert, idealerweise ausgelöscht, und in einem zweiten Teilbereich 32 verstärkt, idealerweise verdoppelt, ist. Die beiden Teilbereiche 31, 32 sind idealerweise gleich groß.

Vor dem Einschalten des Spulenstroms wird die Magnetisierungsrichtung des Spulenkerns 22 von dem Permanentmagneten 23 bestimmt, so dass der Spulenkern 22 von dem Permanentmagneten 23 in den ersten Teilbereich 31 des Antriebsraums 21 hineingezogen, jedoch von dem entgegen gesetzten polarisierten Permanentmagneten 24 aus dem zweiten Teilbereich 32 des Antriebsraums 21 herausgedrückt wird. Vor dem Einschalten eines Spulenstroms ergibt sich deshalb die in Figur 5 dargestellte Position des Spulenkerns 22.

Durch das Einschalten des Spulenstroms entsteht in dem zweiten Teilbereich 32 des Antriebsraums 21 ein erhöhtes Gesamtfeld, das eine Umkehrung der Magnetisierung des weichmagnetischen Spulenkerns 22 bewirkt. Als Folge dieser Umkehrung der Magnetisierungsrichtung des Spulenkerns 22 wird der Spulenkern 22 in den zweiten Teilbereich 32 des Antriebsraums 21 hineingezogen und so aus der in Figur 5 dargestellten Position in eine zweite Position bewegt. Der damit verbundene Hub des Spulenkerns 22 kann für eine Einstichbewegung genutzt werden.

Durch eine Umkehrung der Richtung des Stroms durch die Spule 20 lassen sich die in Figur 7 dargestellten Verhältnisse des Gesamtfeldverlaufs umkehren, so dass sich in dem ersten Teilbereich 31 des Antriebsraums 21 durch konstruktive Überlagerung ein erhöhtes Gesamtfeld und in dem zweiten Teilbereich 32 des Antriebsraums 21 durch destruktive Überlagerung ein reduziertes Gesamtfeld ergibt. Eine derartige Änderung des Gesamtfeldes bewirkt eine erneute Änderung der Magnetisierungsrichtung des Spulenkerns 22, so dass der Spulenkern in den ersten Teilbereich 31 des Antriebsraums 21 zurückgezogen wird und wieder in die erste Position gelangt.

Die Länge des Spulenkerns 22 ist kleiner als die Länge des von der ersten und der zweiten Magnetfeldquelle, also den Permanentmagneten 23, 24, umgebenen Antriebsraums 21, bevorzugt mindestens 10 % kürzer. In diesem Zusammenhang ist unter dem Spulenkern 22 nur die Länge eines weichmagnetischen Teils zu verstehen. Eventuelle Teile, die an einen weichmagnetischen Teil anschließen, aber selbst nicht magnetisch sind, beispielsweise eine Schubstange aus Kunststoff, sind diesbezüglich nicht als Spulenkern anzusehen.

Das anhand der Figuren 4 bis 7 beschriebene Antriebsprinzip kann in entsprechender Weise auch eingesetzt werden, indem die beiden Permanentmagnete 23, 24 durch Spulen mit einander entgegen gesetztem Wicklungssinn und die Spule 20 durch einen Permanentmagneten geeigneter Stärke ersetzt werden.

Bei dem in Figur 4 dargestellten Ausführungsbeispiel ist der Spulenkern 22 in einem Gleitrohr 25 angeordnet. Das Gleitrohr 25 wird mit Zentrierringen 26 in dem Antriebsraum 21 innerhalb des weichmagnetischen Rückschlusses 28 zentriert, der von einem Eisenrohr gebildet wird, das die Polschuhe 27, die Permanentmagnete 23, 24 sowie die Spule 20 und den Antriebsraum 21 umgibt. Auf das Gleitrohr 25 sind weichmagnetische Polschuhe 27 und zwischen den Polschuhen 27 die Permanentmagnetringe 23 und 24 mit entgegen gesetzter Polarisationsrichtung aufgesteckt.

Bei dem anhand der Figuren 4 bis 7 beschriebenen Ausführungsbeispiel erzeugt der Permanentmagnet 23 eine magnetische Haltekraft, die der von dem Permanentmagneten 24 und der Spule 20 erzeugten Antriebskraft entgegen gerichtet ist. Die Spule 20 ist bei diesem Ausführungsbeispiel sowohl Auslösemittel als auch Antriebsmittel. Mit der Spule 20 als Auslösemittel lässt sich die von dem Permanentmagneten 23 erzeugte Haltekraft, nämlich dessen Magnetfeld, soweit reduzieren, dass die Lanzette 8 unter Einwirkung der von dem Antriebsmittel, nämlich dem Permanentmagneten 24 und der Spule 20, erzeugten Antriebskraft in Richtung auf die Hautoberfläche beschleunigt wird.

Ebenso wie bei der anhand der Figuren 1 bis 3 beschriebenen Lanzettenvorrichtung ist auch bei dem anhand der Figuren 4 bis 7 beschriebenen Ausführungsbeispiel die Spule 20 über Strompuffer und/oder Spannungswandler an eine interne Stromquelle der Lanzettenvorrichtung, beispielsweise eine Batterie, angeschlossen, so dass mit handelsüblichen Batterien ein intensiver Strompuls erzeugt werden kann. Für eine möglichst schnelle Einstich- und Rückführbewegung ist es günstig, Änderungen der an der Spule 20 anliegenden Spannung möglichst rasch vorzunehmen, also Spannungspulse mit einem rechteckigen Spannungsprofil zu verwenden. Die Spannungsversorgung der Spule 20 liefert deshalb Spannungspulse mit rechteckigen Flanken. Besonders günstig sind Rechteckspannungen, da durch den Spannungswechsel eines Rechteckspannungspulses die Rückführbewegung der Lanzette eingeleitet werden kann.

### Bezugszeichenliste

- 1: Lanzettenvorrichtung
- 2: Lanzettenantrieb
- 3: Schubstange
- 4: Antriebsfeder
- 5: Permanentmagnet
- 6: Spule
- 7: Lanzettenhalter
- 8: Lanzette
- 10: Führung
- 11: Spulenkern
- 12: Ankerplatte
- 13: Begrenzungselement
- 14: Rückholfeder
- 15: Stützabschnitt der Schubstange
- 16: Polschuh
- 20: Spule
- 21: Antriebsraum
- 22: Spulenkern
- 23: Permanentmagnet
- 24: Permanentmagnet
- 25: Gleitrohr
- 26: Zentrierringe
- 27: Polschuhe
- 28: magnetischer Rückschluss
- 31: Teilbereich des Antriebsraums
- 32: Teilbereich des Antriebsraums

## Patentansprüche

1. Lanzettenvorrichtung, mit der eine Lanzette (8) entlang eines Einstichweges zum Erzeugen einer Einstichwunde in einer Hautoberfläche, insbesondere zum Gewinnen von Körperflüssigkeit für Diagnosezwecke, verschiebbar ist, umfassend
einen Lanzettenantrieb (2) mit einem Antriebsmittel (20, 24) zum Erzeugen einer Antriebskraft für eine Einstichbewegung der Lanzette (8) entlang des Einstichweges in Richtung auf die Hautoberfläche, wobei der Lanzettenantrieb (2) einen Permanentmagneten (23) umfasst, mit dem eine der Antriebskraft entgegen gerichtete magnetische Haltekraft erzeugbar ist, und
der Lanzettenantrieb (2) ferner Auslösemittel (20) umfasst, mit denen die Haltekraft soweit reduzierbar ist, dass die Lanzette (8) unter Einwirkung der von dem Antriebsmittel (20, 24) erzeugten Antriebskraft in Richtung auf die Hautoberfläche beschleunigt wird,
**dadurch gekennzeichnet, dass**
das Antriebsmittel (20, 24) einen weiteren Permanentmagneten (24) umfasst, der dem die Haltekraft erzeugenden Permanentmagneten (23) entgegengesetzt polarisiert angeordnet ist.

2. Lanzettenvorrichtung nach Anspruch1, **dadurch gekennzeichnet, dass** die Auslösemittel eine Spule (20) umfassen, mit der ein Magnetfeld erzeugbar ist, das die Haltekraft des Permanentmagneten (23) zumindest teilweise, vorzugsweise vollständig, kompensiert.

3. Lanzettenvorrichtung nach Anspruch 2, **dadurch gekennzeichnet, dass** der Permanentmagnet (23) hinsichtlich der Spule (20) ortsfest angeordnet ist.

4. Lanzettenvorrichtung nach einem der Ansprüche 2 bis 3, **dadurch gekennzeichnet, dass** die Spule (20) an eine interne Stromquelle der Lanzettenvorrichtung über einen Strompuffer und/oder Spannungswandler zum Erzeugen leistungsstarker Strompulse angeschlossen ist.

5. Lanzettenvorrichtung nach einem der Ansprüche 1 bis 4, **dadurch gekennzeichnet, dass** die Permanentmagnete (23, 24) einen Antriebsraum (21) umschließen, in dem ein Spulenkern (22) beweglich ist, dessen Bewegung im Betrieb als Einstich- und Rückführbewegung auf die Lanzette (8) übertragen wird.

6. Lanzettenvorrichtung nach Anspruch 5, **dadurch gekennzeichnet, dass** die Permanentmagnete (23, 24) als Magnetringe ausgebildet sind.

7. Lanzettenvorrichtung nach einem der vorhergehenden Ansprüche, **dadurch gekennzeichnet, dass** das von dem weiteren Permanentmagneten (24) des Antriebsmittels (20, 24) erzeugte Magnetfeld in einer ersten Phase einer Einstichbewegung, in der die Lanzette (8) von der Antriebskraft in Richtung auf die Hautoberfläche beschleunigt wird, durch konstruktive Überlagerung mit einem weiteren Magnetfeld, das mittels eines Spulenstroms erzeugt wird, verstärkt wird.

## Claims

1. A lancet device by means of which a lancet (8) is displaceable along a puncture path for producing a puncture wound in a skin surface, in particular for obtaining bodily fluid for diagnosis purposes, comprising
a lancet drive (2) with a drive means (20, 24) for producing a driving force for a puncture movement of the lancet (8) along the puncture path towards the skin surface,
wherein the lancet drive (2) comprises a permanent magnet (23) by which a magnetic retention force counter to the driving force is producible, and
the lancet drive (2) further comprising trigger means (20) by which the retention force is reducible to the extent that the lancet (8) is accelerated towards the skin surface under the action of the driving force produced by the drive means (20, 24),
**characterised in that**
the drive means (20, 24) comprises an additional permanent magnet (24) which is arranged oppositely polarised to the permanent magnet (23) generating the retention force.

2. The lancet device according to claim 1, **characterised in that** the trigger means comprise a coil (20) by which a magnetic field is producible that compensates for the retention force of the permanent magnet (23) at least in part, preferably entirely.

3. The lancet device according to claim 2, **characterised in that** the permanent magnet (23) is arranged stationarily with respect to the coil (20).

4. The lancet device according to any of claims 2 to 3, **characterised in that** the coil (20) is connected to an internal power source of the lancet device by means of a power buffer and/or voltage transformer for generating powerful current pulses.

5. The lancet device according to any of claims 1 to 4, **characterised in that** the permanent magnets (23, 24) enclose a drive chamber (21) in which a coil core (22) is movable, the movement of which is transmitted to the lancet (8) as a puncture and restoring movement during operation.

6. The lancet device according to claim 5, **characterised in that** the permanent magnets (23, 24) are formed as magnetic rings.

7. The lancet device according to any of the preceding claims, **characterised in that** the magnetic field produced by the additional permanent magnet (24) of the drive means (20, 24) is reinforced in a first phase of a puncture movement, in which the lancet (8) is accelerated towards the skin surface by the driving force, by constructive superposition with an additional magnetic field, produced by means of a coil current.

## Revendications

1. Dispositif à lancette à l'aide duquel une lancette (8) peut être déplacée le long d'une voie de ponction pour réaliser une incision sur une surface cutanée, en particulier pour extraire un liquide corporel à des fins de diagnostic, comprenant
un entraînement de lancette (2) avec un moyen d'entraînement (20, 24) pour générer une force d'entraînement pour un mouvement d'incision de la lancette (8) le long de la voie de ponction en direction de la surface cutanée,
l'entraînement de lancette (2) comportant un aimant permanent (23), avec lequel une force de retenue magnétique dirigée contre la force d'entraînement peut être générée et l'entraînement de lancette (2) comporte également des moyens de déclenchement (20), avec lesquels la force de retenue peut être réduite de telle sorte que, sous l'influence de la force d'entraînement générée par le moyen d'entraînement (20, 24), la lancette (8) soit accélérée en direction de la surface cutanée,
**caractérisé en ce que**
le moyen d'entraînement (20, 24) comporte un aimant permanent (24) supplémentaire, qui est disposé de manière inversement polarisée par rapport à l'aimant permanent (23) générant la force de retenue.

2. Dispositif à lancette selon la revendication 1, **caractérisé en ce que** les moyens de déclenchement comportent une bobine (20), qui permet de générer un champ magnétique, qui compense au moins partiellement, de préférence entièrement, la force de retenue de l'aimant permanent (23).

3. Dispositif à lancette selon la revendication 2, **caractérisé en ce que** l'aimant permanent (23) est disposé de manière stationnaire par rapport à la bobine (20).

4. Dispositif à lancette selon l'une des revendications 2 à 3, **caractérisé en ce que** la bobine (20) est reliée à une source de courant interne du dispositif à lancette par l'intermédiaire d'un tampon de courant et/ou d'un transformateur de tension pour générer des pulsions de courant performantes.

5. Dispositif à lancette selon l'une des revendications 1 à 4, **caractérisé en ce que** les aimants permanents (23, 24) entourent une chambre d'entraînement (21), dans laquelle est mobile un noyau de bobine (22), dont le mouvement en fonctionnement est transféré en tant que mouvement de ponction et de retrait sur la lancette (8).

6. Dispositif à lancette selon la revendication 5, **caractérisé en ce que** les aimants permanents (23, 24) sont constitués sous la forme d'anneaux magnétiques.

7. Dispositif à lancette selon l'une des revendications précédentes, **caractérisé en ce que** pendant une première phase d'un mouvement de ponction au cours de laquelle la lancette (8) est accélérée par la force d'entraînement dans la direction de la surface cutanée, le champ magnétique généré par l'aimant permanent (24) supplémentaire du moyen d'entraînement (20, 24) est renforcé par superposition constructive avec un champ magnétique supplémentaire, qui est généré à l'aide d'un courant de bobine.
